# EUROPEAN PATENT APPLICATION

(11) **EP 0 766 218 A1**
(43) Date of publication of application: **02.04.1997**
(21) Application number: 96906972.3
(22) Date of filing: 01.03.1996
(51) Int. Cl.: G09B 21/04, A61F 11/04, A61F 9/08, A61B 5/16

(54) **METHOD OF TRANSMITTING INFORMATION TO A HUMAN NERVOUS SYSTEM**

(30) Priority: 17.04.1995 RU 95105733
(71) Applicant: Fingerov, Gavriil Mikhailovich, Moscow, 113055 (RU); Jurin, Sergei Vladimirovich, Moscow (RU); Paryshev, Vladimir Viktorovich, Moscow, 129339 (RU)
(72) Inventor: Fingerov, Gavriil Mikhailovich, Moscow, 113055 (RU); Jurin, Sergei Vladimirovich, Moscow (RU); Paryshev, Vladimir Viktorovich, Moscow, 129339 (RU)
(74) Representative: BOVARD AG - Patentanwälte
(86) International application number: RU9600050
(87) International publication number: WO9633481

(57) **Abstract**

The invention relates to a method of conveying information to the nervous system of a person without using audio-visual channels of perception. The method includes coding information into tactually perceived signals and teaching a person to identify these signals, the initial information being disintegrated into separate streams and each of the streams being coded as a sequence of discreet codes, the codes of every stream being transmitted to separate fixed points of a person skin, and during the process of teaching the person acquires the capacity to synthesise the received signals into the final information adequate to the initial one.

The initial information is being coded with electrical signals with different frequency and/or amplitude characteristics of voltage and/or current intensity, mechanical vibration signals and different amplitude and/or frequency and/or localisation and thermal signals, the duration of codes and time intervals between them being varied and the extent of disintegration of the initial information is being controlled.

The invention makes possible to create the systems of artificial vision and hearing and also to create an absolutely new system of informational interchange between a machine and a person which can be applied for non-audio-visual monitoring of the work of a machine and its controlling with the purpose of crating non-audio-visual system of communication, creating adaptive artificial limbs, etc. The distinctive feature of the invention is non-invasive character of its realisation, i.e. this method does not require surgical invasion which makes it possible to apply the invention to both the healthy and the disabled people, needing rehabilitating devices.

## Description

### Field of the Invention

The invention relates to a method of conveying information to the nervous system of a person without using visual and audible channels of perception.

The invention can be applied to the systems of artificial sight and to systems of controlling different devices including artificial limbs, etc.

### Prior Art

It is known that there are ways of conveying information to the nervous system without using audial and visual channels of perception based, for example on using the walking-stick-probe by the blind (for example, see "Radio" magazine ("the Radio") #9, 1980, page 15-16), such ways are not perfect, because they do not give a blind person an opportunity to orientate himself to a situation with required freedom.

There exist better methods of conveying information to the nervous system of a person including coding the initial information into signals to be perceived tactually and teaching a person to identify these signals (for example, see certificate USSR #955920 Int.Cl. A61F9/08, 1980)

The above stated method is the closest analogue of the invention in essence and scope of features.

This method can only be used for the systems of artificial sight. However, even in this field it cannot ensure proper orientation in space because it only enables to identify the illumination of one fragment of an image at a time.

### Summary of Invention

The present invention makes it possible to solve the problem of perception of information about individual objects or the environment as a whole without using audial and visual channels of perception.

The solution of the given problem can be achieved due to the fact that according to the method of conveying information to the nervous system of a person by coding the initial information into signals to be perceived tactually and by teaching a person to identify the initial information, the initial information is disintegrated into separate streams and each of the streams is coded as a sequence of discreet codes, the codes of each stream being conveyed to separate fixed points of a person's skin and during the process of teaching a person acquires the capability of synthesising the signals received into the final information that is adequate to the initial one.

The initial information is being coded with electric signals with different frequency and/or amplitude characteristics of voltage and/or current intensity, mechanical vibration signals with different amplitudes and/or frequencies, or thermal signals.

At the same time the duration of the codes and the time intervals between them is being changed and the extent of disintegration of initial information is controlled.

The above-stated terms mean as follows:
- the nervous system of a person is a scope of structures in a person's organism combining the activities of all the organs and systems and ensuring functioning of the organism as a whole in its constant interaction with the environment (see "Bolshaya Sovetskaya Entsiclopedia" ("The Great Soviet Encyclopaedia"), volume 17, page 505, 1974, Moscow, "Sovetskaya Entsiclopedia" Publishing House).
- Coding the initial information is transformation of light, acoustic and other information about the environment, texts, etc. into a system reflecting this information adequately.
- Signals perceived tactually are signals to which the skin of a person is sensible.
- A discreet code is a signal that can be separately reproduced tactually.
- Electric signal is an impulse of electric current and the initial information can be coded depending on changing a frequency and/or amplitude of current intensity and/or voltage of impulses.
- Thermal signal is a thermal influence on the skin of a person that can be perceived tactually.
- Disintegration of the initial information is dividing the initial information into blocks, each of them having a value necessary for turning the initial information into the final one, so that one to one correspondence between the fragments extracted from the initial and final information is established and the final information is made accessible to the nervous system of a person due to the scope of signals perceived tactually.

Some examples of the invention as applied to practice.

### Detailed Description of Preferable Embodiments

### Example 1

A method of conveying textual information to the nervous system. In this case the text (the initial information) is to be divided into separate streams, i.e. words and/or letters.

Five vibrostimulators can be fastened onto the body of a person (acceptor), for example to his shoulder at a distance of 20-30 mm.

One code is a combination of influences created by all the five stimulators simultaneously during the given time that can be equal to 0.2 - 1.5 s. Letting symbol 1 represent a switched on stimulator and symbol 0 represent a "silent" stimulator, a number of vibrostimulator being the order of its designation in a five-digit record, the letters of the alphabet can be coded, for example, in this way.

| | | | |
|---|---|---|---|
| a - 10000 | c - 11010 | e - 1110 | g - 01000 |
| b - 11000 | d - 11011 | f - 01010 | h - 01001 |

An acceptor should be first taught to identify every letter. On the average it takes the acceptor 14-28 hours to be taught to identify the letters of the alphabet; after that the acceptor is being taught to synthesise words with letters being conveyed. Words conveyance is to be separated with a pause, for example, equal to two durations of one code influence. Fluency of reading can be achieved in 3 - 4 days.

### Example 2

In case a sign is coded as an electroimpulse one code may represent electroinflunce in the mode of transmitting electroimpulses of different frequencies, for example, being transmitted simultaneously to three contacts (a code group) situated on the surface of a body. A code is to be synthesised from electroimpulses with frequencies that can be one-to-one identified by the nervous system, for example, 20, 50 and 120 Hertz.

In this case the three frequencies and one silent contact form 3 to the 4th power not repeating combinations, i.e. 81 sign can be coded.

In this case four contacts can be fastened to the surface of an acceptor's body, for example, to his shoulder, for conveying textual information, three of them being used for transmitting influence and one contact being common for all the contact group. The contacts of the code group are placed at a distance of 7 - 9 mm. A combination of influence frequencies and the place of transmitting them can be achieved so that it could correspond to each letter of the alphabet. If a code can be written as a sequence of numbers the order of which corresponds to the number of a contact in the contact code group and the value of a number corresponds to the degree of influence.
0 - the absence of influence
1 - frequency is 20 Hertz
2 - frequency is 50 Hertz
3 - frequency is 120 Hertz
then the figures and the letters of the alphabet can be coded as follows:

| | | | | |
|---|---|---|---|---|
| 1 - 001 | 6 - 030 | a - 101 | f - 130 | k - 131 |
| 2 - 002 | 7 - 011 | b - 111 | g - 121 | l - 132 |
| 3 - 003 | 8 - 012 | c - 121 | h - 122 | m - 133 |
| 4 -010 | 9 - 013 | d -113 | i - 123 | n - 200 |
| 5 - 020 | 0 - 021 | e - 120 | j - 130 | o - 201, etc. |

The duration of transmitting one code with the help of electroinfluence equals 0.2 - 1.5 s.

An acceptor is first being taught to identify every letter and figure. On the average it takes an acceptor 14 - 28 hours to be taught to identify letters and figures and the acceptor is being taught to synthesise words with the letters being transmitted and to reproduce the sense of conveyed information. The conveyance of words is to be separated by a pause equal to two durations of one code influence. Fluency of "reading" can be achieved in 10 - 20 days.

### Example 3

The invention can be used for conveying information to the nervous system from a measuring instrument or a group of instruments. In this case every instrument has its own contact group corresponding to it. For example, these contact groups can be placed on one's wrist as a bracelet, etc.

There has been created a model of a working place of an operator of a steam-generator. The instruments were to measure pressure in the evaporator, temperature of steam and expenditure of combustible gas. The operator's duty was to control the combustible gas expenditure in order to maintain the steam temperature and its pressure within the given limits.

The contact group of a pressure sensor was fastened to the right wrist, the contact group of a temperature sensor was fastened to the left wrist and the data concerning combustible gas expenditure were conveyed to the right forearm, i.e. the initial information in this case was the indication of the above-mentioned sensors and the flowmeter.

The operator was first being taught to identify the codes from the sensors and after that the tests were carried out on the model of the controlling object. It took the operator 3 days to be taught to control the work of the model while the information about its condition was being transmitted with the help of electroimpulse influence and after that he could re-establish correctly the operating range of temperature and pressure if gas expenditure was changing.

The conveyance of information can be carried out in a digital way that is analogous to the above mentioned one or in analogous way, when the intensity of influence should correspond to the value being transmitted.

In this case, for example, the intensity of influence in the form of a current intensity of electroimpulse should correspond to the value of the parameter measured.

The scale of pressure variation within the range of 1.5 - 3 atmospheres was juxtaposed to the current intensity equal to 2 - 6 mA according to the linear law, the impulse duration being equal to 0.4 ms and the frequency of their transmitting being equal to 80 Hertz. The dependence of the value of the parameter coded on the intensity of code influence corresponding to it can be expressed in the power law, exponential law, etc.

The operator was being taught to orient oneself to this scale and to assess the value of pressure from the intensity of an analogous signal. After training the identification of value of measured parameter was assessed by the operator with an accuracy of 5 - 10%

### Example 4

Coding information can be carried out with the help of thermal signals. For example, it can be carried out according to scheme that is analogous to coding information with the help of vibration influence. In this case semiconductor thermoactive elements can be fastened to the surface of a man's body, for example, on his forearm. The elements work in the mode of heat conveyance to the contact surface on the acceptor's body or heat withdrawal from it depending on the polarity of the currency conveyed to it. Five thermoactive semiconductor elements are fastened to the acceptor's body, for example, to the surface of the forearm, the elements being placed at a distance of 20-30 mm. from each other. One code is a combination of influences made by all the five elements simultaneously during the given time equals approximately to 0.2 - 1.5 s. If the elements sensing heat emission, i.e. "warm" is labelled as "0" and the element sensing heat loss i.e. "cold" is labelled as the symbol "1", an element number being the order of its labelling in the 5-digit records, then the letters of an alphabet can be coded for example in this way:

| | | | |
|---|---|---|---|
| a - 10000 | c - 11010 | e - 11110 | g - 01000 |
| b - 11000 | d - 11011 | f - 01010 | h - 01001, etc. |

An acceptor is first being taught to identify every letter. On the average it takes an acceptor 16 - 30 hours to be taught to identify the letters of the alphabet, and after that the acceptor is taught to synthesise words with the letters being conveyed. The conveyance of words should be separated by a pause equal, for example, to two durations of one code influence. The fluency of "reading" can be achieved in 5 - 8 days.

### Example 5

The invention can be applied to the system capable of giving the people with defects of hearing an opportunity to perceive and analyse sounds and speech.

In this case sounding (the initial acoustic information) can be perceived by a recording and processing device. The initial information is being disintegrated (resolved) into a discreet range of frequencies and turned into a spectral signal. The spectral signal is a scope of simultaneous secondary signals, each of which corresponds to its frequency, the value of each secondary signal being proportional to the presence of the correspondent frequency in the second being transmitted at a given moment of time.

It is sufficient, for example, to resolve the initial acoustic information into about 20 secondary signals for identifying speech and a person speaking. In this case the contact group consist of 20 contacts placed on the surface of the body. Each of the contacts corresponds to its secondary signal, i.e. to the frequency it represents.

A spectral signal is divided into fragments representing successive intervals of sounding, for example, their duration may be equal to 15 - 20 ms.

In each fragment every secondary signal has constant intensity, equal to intensity averaged over the duration of the fragment.

Every output signal is being generated in the device from every secondary signal in the form, for example, of an electroimpulse signal the intensity of which corresponds to the intensity of the given secondary signal. The secondary signals are being transmitted simultaneously to the corresponding contact of the code group and in this way form a code perceived by the nervous system.

In this case the code of a fragment is a scope of electroimpulse influences transmitted simultaneously to the corresponding contacts of the group. In the code the frequency existing in the transmitted sound fragment is represented by the place of the contact on the surface of the body and its intensity of electroinfluence applied to contact.

The conveyance of information in taking place by successive transmitting of codes to the nervous system. Every next code is being transmitted at a time interval equal to the duration of a fragment.

Several contact groups can be placed on the surface of a body for increasing the capacity of the nervous system to identify the fragments coded in this way. The first fragment should be transmitted to the first group, the second fragment should be transmitted to the second group, the third fragment should be transmitted to the third group and the fourth fragment, for example, should be transmitted to the first group again. If more time is needed for restoring the perceived capacity of the body the number of groups should be increased, for example, to 10 - 20.

A person is being taught to identify sounds and their rhythm and also to identify the sounding of letters and to synthesise the sense of speech from arising sensation.

### Example 6

The invention can be also applied to conveying information about an image to the nervous system. To this end a videoimage can be received, for example, with the help of a changing matrix for registration of optical image and the information about it can be transmitted to the device. In the device the information about the image can be disintegrated into fragments so that this disintegration is equal to dividing the image itself into fragments of the given value. In the device every fragment of the image cam be transformed into a scope of data in which an average illumination of the fragment of the image and its coordinates in the picture are represented.

An output signal is being formed for every fragment on the basis of the scope of data, for example, in the form of an electroimpulse influence. In every output signal the illumination of the fragment is represented by its intensity and the place of the fragment is represented by a contact in the code group to which this influence is transmitted. If the position of the fragment is coded with a contact in the code group and frequency, then information about several fragments can be transmitted to the contact. In this case the number of contacts of the code group will be less that the number of the fragments after dividing the image.

A code is a scope of output signals transmitted to the contacts of the code group simultaneously.

Transmitting the codes about the fragments of the image can be carried out with a frequency, determined by the speed of transmitting the image from the charging matrix to the device with the sequence of stills.

In the process of teaching a person the pattern of transmitting codes should be adjusted in such a way that the nervous system of a person could react to the signals corresponding to every individual fragment of the image separately from other signals being transmitted from other fragments, i.e. so that every fragments of the image is perceived separately. For this purpose testing is used with adjusting in the form of feedback from a person; such type of dividing and such type of coding should be reached that signals from every fragment of dividing of an image could be perceived by a person separately. A person is being taught to find his way in space with the help of a tactile image appearing as a result of application of codes. The changing matrix for recording optical image should be fastened, for example, onto the head of a person who may hold in his hand a pointy source of light and change its position. He is being taught find this way in space using appearing sensations and the training should continue till automatism appears, when the person can take in his hand the source of light moving before him and after that the training should be expanded till a person can move in space independently.

As a result of teaching a person acquires capacity for identifying the shape of objects and their place in space and also to tell the features of one object from the other, to identify letters and to read.

If implementation about an image could be received in invisible, e.g. infrared, spectre, a person can be taught to orient himself in space in the dark without using a visual channel of perception like in instruments of night vision.

### Example 7

The invention can be applied to creating artificial limbs which are referential to the nervous system of a person. For this purpose an artificial limb, for example, an artificial leg, is equipped with the sensors, which generate signals about the value of pressure of a foot on a surface and about the position of the parts of the artificial limb. The signals from the sensors are being transmitted to the device and transformed into output signals.

Generating such signals can be organised in the form of signs or analogues.

On the basis of the codes perceived by the nervous system a person can be taught to appreciate the position and condition of an artificial limb.

An analogous method can be applied to creating a feedback with the operator of a manipulator, imitating the functions of a hand. In this case the application of this method makes it possible to report to the operator about for example the intensity of compression of a sample and to receive an additional information about the order of its part and their location.

### Industrial Appliance.

As it follows from the above-stated, the invention makes it possible to convey information presented in a visual or audial way without using visual and audial channels of perception in such a way that the content and the sense of the information conveyed can be synthesised by a person adequately. It makes it possible to create the systems of artificial vision and hearing and also to create absolutely new interface between the machine and the person, which can be applied to the new system of communication, adaptive artificial limbs, etc. The distinctive feature of the invention is non-invasive character of its realisation, i.e. application of this method does not require surgical invasion.

And besides, as appears from the above-stated examples, the implementation of this invention will not cause technological problems.

## Claims

1. A method of conveying information to the nervous system of a person including coding the initial information into tactually perceived signals and teaching a person to identity these signals is characterised by the fact that the initial information is being disintegrated into separate streams, each of the streams is being coded in the form of the sequence of discreet codes, the codes of each stream being transmitted to separate fixed points of a person's skin, and the person is being taught to cultivate the ability to synthesise the received signals into the final information adequate to the initial one.

2. The method of claim 1 wherein the initial information is being coded with electric signals with different frequency and/or amplitude characteristics of voltage and/or current intensity and/or localisation on the surface of the body.

3. The method of claim 1 wherein the initial information is being coded with mechanical vibration signals with different amplitude and/or frequency and/or localisation on the surface of the body.

4. The method of claim 1 wherein initial information is being coded with thermal signals.

5. The method of claim 1 - 4 wherein the duration of the codes and time intervals between them is being varied.

6. The method of claim 1 - 5 wherein the extent of disintegration of the initial information is being controlled.
